(19)

Europäisches Patentamt
European Patent Office
Office européen des brevets

(11) **EP 3 782 553 B1**

(12) **EUROPEAN PATENT SPECIFICATION**

(45) Date of publication and mention
of the grant of the patent:
**01.01.2025 Bulletin 2025/01**

(21) Application number: **18915398.4**

(22) Date of filing: **26.04.2018**

(51) International Patent Classification (IPC):
*A61B 8/08* (2006.01)          *A61B 8/00* (2006.01)

(52) Cooperative Patent Classification (CPC):
**A61B 8/461; A61B 8/5207; G01S 7/52026;
G01S 7/52077; G01S 15/8915;** A61B 8/14;
A61B 8/4405; A61B 8/4427; A61B 8/4472;
A61B 8/4477; A61B 8/467; A61B 8/5269;
G01S 7/5208; G01S 7/52084

(86) International application number:
**PCT/KR2018/004828**

(87) International publication number:
**WO 2019/203383 (24.10.2019 Gazette 2019/43)**

(54) **ULTRASOUND IMAGE DEVICE AND ULTRASOUND IMAGE GENERATION METHOD**

ULTRASCHALLBILDVORRICHTUNG UND ULTRASCHALLBILDERZEUGUNGSVERFAHREN

DISPOSITIF D'IMAGE ULTRASONORE ET PROCÉDÉ DE GÉNÉRATION D'IMAGES
ULTRASONORES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.04.2018 KR 20180044114**

(43) Date of publication of application:
**24.02.2021 Bulletin 2021/08**

(73) Proprietor: **Samsung Medison Co., Ltd.
Hongcheon-gun, Gangwon-do 25108 (KR)**

(72) Inventors:
• **CHO, Seung-woo
Seoul 01761 (KR)**
• **SONG, Jong-keun
Yongin-si, Gyeonggi-do 16950 (KR)**

(74) Representative: **Arnold & Siedsma
Bezuidenhoutseweg 57
2594 AC The Hague (NL)**

(56) References cited:
KR-A- 20180 005 930      KR-B1- 100 947 824
US-A1- 2005 113 694      US-A1- 2007 232 917
US-A1- 2009 016 163      US-A1- 2017 146 643
US-A1- 2018 008 234

• **CHEN CHAO ET AL: "A Prototype PZT Matrix
Transducer With Low-Power Integrated Receive
ASIC for 3-D Transesophageal
Echocardiography", IEEE TRANSACTIONS ON
ULTRASONICS, FERROELECTRICS, AND
FREQUENCY CONTROL, IEEE, USA, vol. 63, no.
1, 1 January 2016 (2016-01-01), pages 47 - 59,
XP011596523, ISSN: 0885-3010, [retrieved on
20151230], DOI: 10.1109/TUFFC.2015.2496580**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

TECHNICAL FIELD

Embodiments of the disclosure relate to an ultrasound imaging apparatus and a method of generating an ultrasound image.

BACKGROUND ART

Medical imaging apparatuses such as an X-ray diagnostic apparatus, a computed tomography (CT) scanner, a magnetic resonance imaging (MRI) apparatus, a nuclear medicine diagnostic apparatus, and an ultrasound imaging apparatus for obtaining at least one image of an internal part (e.g., soft tissue or blood flow) of an object by using ultrasound signals are equipment for obtaining images of internal structures of the object. Medical imaging apparatuses are non-invasive testing devices and capture images of structural details, tissue, and flow of a fluid inside a body, process the images, and display them to a user. A user such as a medical doctor may diagnose a patient's health condition and disease by using a medical image output from a medical image processing apparatus.

Ultrasound medical imaging apparatuses transmit ultrasound signals generated by transducers of a probe to an object and detect information about signals reflected from the object, thereby obtaining at least one image of an internal part, for example, soft tissue or blood flow, of the object.

Furthermore, to achieve more improved image quality, the number of channels needs to be increased.

Increasing the number of channels requires an increase in a thickness of a probe cable, and to solve this problem, analog beamforming is performed by inserting an analog application specific integrated circuit (ASIC) next to transducers in the probe.

When an ultrasound imaging apparatus performs analog beamforming, a control signal from a time delay circuit may be included as noise (e.g., clock harmonic leakage) in ultrasound data.

An example of such an ultrasound imaging apparatus may be found for example in US 2018/008234 A1.

A conventional ultrasound imaging apparatus including an analog beamformer performs beamforming by delaying and summing signals using the same number of capacitors regardless of delay time. The signal-to-noise ratio (SNR) of ultrasound data generated by the conventional ultrasound imaging apparatus is reduced by overlapping noise of a specific frequency. Thus, the conventional medical ultrasound imaging apparatus has a problem in that unclear ultrasound image data is generated.

Accordingly, it is necessary to increase an SNR by performing analog beamforming so that noise does not overlap one another at a specific frequency.

DESCRIPTION OF EMBODIMENTS

TECHNICAL SOLUTION TO PROBLEM

The invention is defined in the independent claims 1, 8 and 9. Provided is a method of generating an ultrasound image including: setting a focal point in an object; transmitting, based on a location of the focal point, ultrasound signals into the object by using a plurality of elements in a transducer; receiving, via the plurality of elements, ultrasound echo signals obtained by reflecting the ultrasound signals from the object; and performing beamforming on the received ultrasound echo signals, wherein the performing beamforming of the received ultrasound echo signals includes: setting, based on the location of the focal point, a plurality of delay times respectively corresponding to the plurality of elements; determining the number of capacitors to be used for time delay, respectively corresponding to the plurality of elements based on the set plurality of delay times, such that a time when a signal is not stored in a capacitor used for the time delay is less than a predetermined time; and respectively performing the beamforming on the received ultrasound echo signals by using the determined number of capacitors.

ADVANTAGEOUS EFFECTS OF DISCLOSURE

Embodiments of the disclosure may provide an ultrasound imaging apparatus and ultrasound image generation method for increasing a signal-to-noise ratio (SNR).

BRIEF DESCRIPTION OF DRAWINGS

FIG. 1 is a block diagram illustrating an ultrasound imaging apparatus according to an exemplary embodiment;
FIGS. 2A, 2B, and 2C are diagrams respectively illustrating an ultrasound imaging apparatus according to an exemplary embodiment;
FIG. 3 is a circuit diagram of an analog time delay circuit according to an exemplary embodiment.
FIG. 4 illustrates signals used for a conventional ultrasound imaging apparatus to control an operation of a time delay circuit.
FIG. 5 illustrates noise generated according to a delay time in a time delay circuit of a conventional ultrasound imaging apparatus.
FIG. 6 illustrates a signal and noise generated when a conventional ultrasound imaging apparatus performs beamforming.
FIG. 7 is a flowchart of a method, performed by an ultrasound imaging apparatus, of performing beamforming, according to an exemplary embodiment.
FIGS. 8A through 8G respectively show that an ultrasound imaging apparatus perform time delays by using capacitors, according to an exemplary embodiment.

FIG. 9 illustrates noise generated according to a delay time in a time delay circuit according to an exemplary embodiment.

FIG. 10 illustrates a signal and noise generated when an ultrasound imaging apparatus performs beamforming, according to an exemplary embodiment.

FIG. 11 illustrates a correlation among delay time, noise, and the number of capacitors used when an ultrasound imaging apparatus performs beamforming, according to an exemplary embodiment.

FIG. 12 is a flowchart of a method, performed by an ultrasound imaging apparatus, of performing beamforming, according to an exemplary embodiment.

FIG. 13 is a flowchart of a method, performed by an ultrasound imaging apparatus, of performing beamforming, according to an exemplary embodiment.

BEST MODE

According to an aspect of the present disclosure, a method according to claim 1 is provided.

The number of capacitors corresponding to each of the plurality of elements may be determined by comparing each of the set plurality of delay times with a preset reference time.

The determining of the number of capacitors may include: determining a range corresponding to each of the set plurality of delay times from among a plurality of ranges obtained via classification based on the preset reference time by comparing each of the set plurality of delay times with the preset reference time; and determining the number of capacitors corresponding to the determined range.

The number of capacitors corresponding each of the plurality of elements may be determined by comparing a longest delay time among the set plurality of delay times with each of the set plurality of delay times.

The determining of the number of capacitors may include: classifying the longest delay time into a plurality of ranges based on a preset reference time; determining a range corresponding to a delay time corresponding to each of the plurality of elements from among the plurality of ranges by comparing the delay time with a preset reference time; and determining the number of capacitors corresponding to the determined range.

The preset reference time may be set by applying a predetermined reference ratio to the longest delay time.

The number of a plurality of elements having the same determined number of capacitors may be less than or equal to a predetermined value.

The number of types of a category indicating the determined number of capacitors may be greater than or equal to a predetermined value.

The method may further include: converting the beamformed ultrasound echo signals into ultrasound image signals; and displaying the ultrasound image signals.

According to another aspect of the present disclosure, an ultrasound imaging apparatus according to claim 9 is provided.

The processor may be further configured to determine the number of capacitors corresponding to each of the plurality of elements by comparing each of the set plurality of delay times with a preset reference time.

The processor may be further configured to: determine a range corresponding to each of the set plurality of delay times from among a plurality of ranges obtained via classification based on the preset reference time by comparing each of the set plurality of delay times with the preset reference time; and determine the number of capacitors corresponding to the determined range.

The processor may be further configured to determine the number of capacitors corresponding each of the plurality of elements by comparing a longest delay time among the set plurality of delay times with each of the set plurality of delay times.

The processor may be further configured to: classify the longest delay time into a plurality of ranges based on a preset reference time; determine a range corresponding to a delay time corresponding to each of the plurality of elements from among the plurality of ranges by comparing the delay time with a preset reference time; and determine the number of capacitors corresponding to the determined range.

The processor may be further configured to set the preset reference time by applying a predetermined reference ratio to the longest delay time.

The number of a plurality of elements having the same determined number of capacitors may be less than or equal to a predetermined value.

The number of types of a category indicating the determined number of capacitors may be greater than or equal to a predetermined value.

The processor may be further configured to convert the beamformed ultrasound echo signals into ultrasound image signals, and the ultrasound imaging apparatus may further include a display displaying the ultrasound image signals.

According to another aspect of the present disclosure, a computer program product according to claim 8 is provided.

MODE OF DISCLOSURE

The present specification describes principles of the disclosure and sets forth embodiments thereof to clarify the scope of claims of the disclosure and to allow those of ordinary skill in the art to implement the embodiments of the disclosure. The embodiments of the disclosure may be implemented in various forms.

Like reference numerals denote like elements throughout. The present specification does not describe all components in the embodiments, and common knowledge in the art or the same descriptions of the embodiments will be omitted below. Terms such as "part" and "portion" used herein denote those that may be embodied by software or hardware. According to exemplary embodiments, a plurality of parts or portions may be embodied by a single unit or element, or a single part or portion may include a plurality of elements. Hereinafter, the principles and embodiments of the present disclosure will be described in detail with reference to the accompanying drawings.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

Embodiments will now be described with reference to the accompanying drawings.

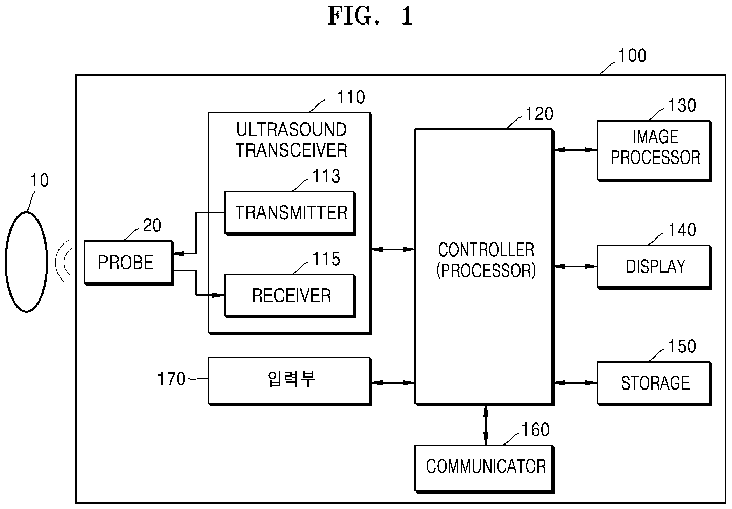

FIG. 1 is a block diagram illustrating a configuration of an ultrasound imaging apparatus 100, i.e., a diagnostic apparatus, according to an exemplary embodiment. Referring to FIG. 1, the ultrasound imaging apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound imaging apparatus 100 may be of a cart-type or a portable-type ultrasound imaging apparatus, that is portable, moveable, mobile, or hand-held. Examples of the portable-type ultrasound imaging apparatus 100 may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound imaging apparatus 100 may be formed in one body (e.g., disposed in a single housing), or the probe 20 and the ultrasound imaging apparatus 100 may be formed separately (e.g., disposed separately in separate housings) but linked wirelessly or via wires. In addition, the ultrasound imaging apparatus 100 may include one or more probes 20 according to embodiments.

The controller 120 may control all operations of the ultrasound imaging apparatus 100. Furthermore, the controller 120 may control an operation of each component of the ultrasound imaging apparatus 100. The controller 120 may be implemented as at least one processor. The processor may be a general-purpose processor (e.g., a central processing unit (CPU), an application processor, or the like). Alternatively, the processor may be manufactured for providing functions of obtaining and displaying an ultrasound image

The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

Each of a plurality of transducer elements of a transducer in the probe 20 may be connected to a channel for processing a received echo signal. Each of a plurality of channels may include a compensator and a time delay circuit. Signals respectively output through the channels may be added together by a summer and then converted into ultrasound data.

The compensator may perform time gain compensation and depth gain compensation of an ultrasound echo signal. The compensator may equally compensate for a difference in amplitudes of echo signals received from objects having different depths. When a distance between an object and each of the transducer elements varies, amplitudes of echo signals respectively received from the transducer elements are not equal to one another. Thus, compensation corresponding to the degree of attenuation needs to be performed on echo signals respectively received from the transducer elements. In particular, the longer an ultrasound wave propagation path, the larger the attenuation of an echo signal and the later the echo signal arrives. Thus, the compensator may compensate for the attenuation by amplifying an amplitude of a later arriving echo signal to the same amplitude as an earlier arriving echo signal.

Because echo signals respectively arrive at the transducer elements at different times, the time delay circuit delay a time to align an earlier arriving echo signal with a later arriving echo signal.

The controller 120 may set a delay time corresponding to each of the transducer elements based on a location of a focal point. The controller 120 may determine a capacitor corresponding to each of the transducer elements based on the set delay time. The controller 120 may beamform a received ultrasound echo signal by using the determined capacitor.

According to an exemplary embodiment, the controller 120 may determine the number of capacitors corresponding to a transducer element by comparing a delay time corresponding to the transducer element with a preset reference time.

According to an exemplary embodiment, the controller 120 may determine a range corresponding to the delay time set for the transducer element from among a plurality of ranges obtained via classification based on the reference time. The controller 120 may determine a range corresponding to the delay time by comparing the delay time with the reference time. The controller 120 may determine the number of capacitors corresponding to the determined range as the number of capacitors corresponding to the transducer element.

According to an exemplary embodiment, the controller 120 may determine the number of capacitors corresponding to a transducer element based on a longest delay time among a plurality of delay times respectively set for the transducer elements. The controller 120 may classify the longest delay time into a plurality of ranges based on the reference time. The controller 120 may determine a range corresponding to a delay time for a transducer element by comparing the delay time with the reference time. The controller 120 may then determine the number of capacitors corresponding to the determined range as the number of capacitors corresponding to the transducer element. The controller 120 may set the reference time by applying a predetermined reference ratio to the longest delay time. For example, the controller 120 may set times that are multiples of a minimum delay time $\Delta t$ as the reference time by applying a multiple of the minimum delay time $\Delta t$ to the longest delay time as the reference ratio. As another example, the controller 120 may set, as the reference time, a multiple of a value obtained by dividing the longest delay time by the number of capacitors that are to be used for a time delay.

According to an exemplary embodiment, the controller 120 may determine the number of capacitors used for a time delay so that noise included in echo signals output from the time delay circuit does not overlap one another. For example, the controller 120 may increase the number of capacitors corresponding to one of the transducer elements having the same number of capacitors used for a time delay. As another example, the controller 120 may determine the number of capacitors corresponding to each of the transducer elements such that the number of transducer elements delaying a time by using the same number of capacitors is less than or equal to a predetermined value. As another example, the controller 120 may determine the number of capacitors used for a time delay such that a signal-to-noise ratio (SNR) of ultrasound data at frequencies where a noise level is maximum is less than or equal to a predetermined value. As another example, the controller 120 may determine the number of capacitors so that the number of capacitors corresponding to each of the transducer elements may be of various types. In other words, the controller 120 may determine the number of capacitors corresponding to each of the transducer elements such that the number of types of a category indicating the number of capacitors corresponding to each of the transducer elements is greater than or equal to a predetermined value.

The image processor 130 may generate an ultrasound image by using ultrasound data generated from the ultrasound receiver 115.

The display 140 may display a generated ultrasound image and various pieces of information processed by the ultrasound imaging apparatus 100. The ultrasound imaging apparatus 100 may include two or more displays 140 according to the present exemplary embodiment. The display 140 may include a touch screen in combination with a touch panel.

The controller 120 may control the operations of the ultrasound imaging apparatus 100 and flow of signals between the internal elements of the ultrasound imaging apparatus 100. The controller 120 may include a memory for storing a program or data to perform functions of the ultrasound imaging apparatus 100 and a processor and/or a microprocessor (not shown) for processing the program or data. For example, the controller 120 may control the operation of the ultrasound imaging apparatus 100 by receiving a control signal from the input interface 170 or an external apparatus.

The ultrasound imaging apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound imaging apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound imaging apparatus 100 may process the data of the external apparatus in response to the control signal of the controller 120 received via the communicator 160.

A program for controlling the ultrasound imaging apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound imaging apparatus 100, input and/or output ultrasound data, ultrasound images, applications, etc.

The input interface 170 may receive a user's input to control the ultrasound imaging apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

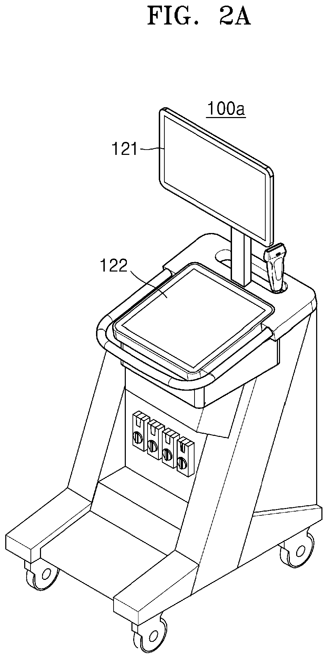

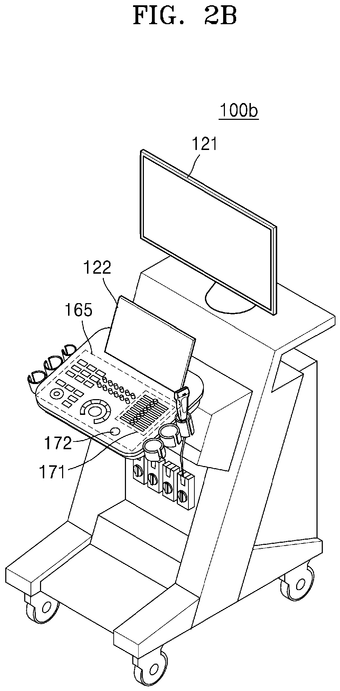

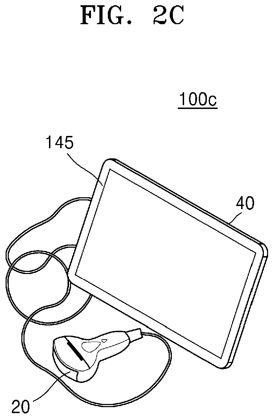

An example of the ultrasound imaging apparatus 100 according to the present exemplary embodiment is described below with reference to FIGS. 2A, 2B, and 2C.

FIGS. 2A, 2B, and 2C are diagrams illustrating ultrasound imaging apparatus according to an exemplary embodiment.

Referring to FIGS. 2A and 2B, the ultrasound imaging apparatus 100 may include a main display 121 and a sub-display 122. At least one among the main display 121 and the sub-display 122 may include a touch screen. The main display 121 and the sub-display 122 may display ultrasound images and/or various information processed by the ultrasound imaging apparatus 100. The main display 121 and the sub-display 122 may provide graphical user interfaces (GUI), thereby receiving user's inputs of data to control the ultrasound imaging apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound imaging apparatus 100 may control the display of the ultrasound image on the main display 121 by using the input control data.

Referring to FIG. 2B, the ultrasound imaging apparatus 100 may include a control panel 165. The control panel 165 may include buttons, trackballs, jog switches, or knobs, and may receive data to control the ultrasound imaging apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound imaging apparatus 100 may keep displaying a frame image at that time point.

The buttons, trackballs, jog switches, and knobs included in the control panel 165 may be provided as a GUI to the main display 121 or the sub-display 122.

Referring to FIG. 2C, the ultrasound imaging apparatus 100 may include a portable device.

An example of the portable ultrasound imaging apparatus 100 may include, for example, smart phones including probes and applications, laptop computers, personal digital assistants (PDAs), or tablet PCs, but an exemplary embodiment is not limited thereto.

The ultrasound imaging apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound imaging apparatus 100, and a GUI.

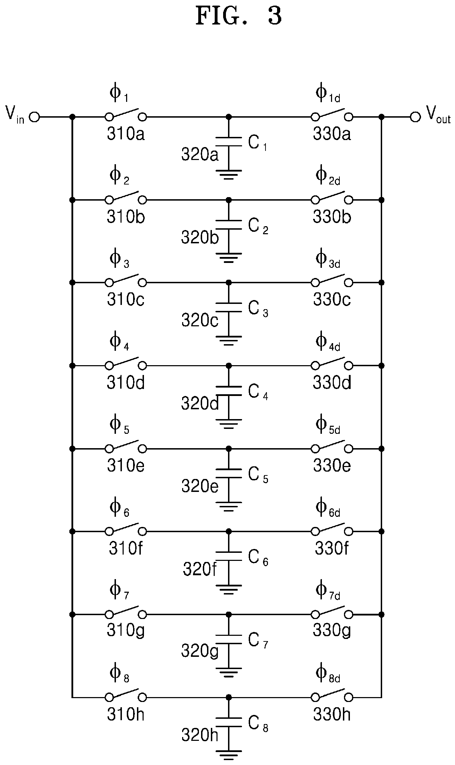

FIG. 3 is a circuit diagram of an analog time delay circuit according to an exemplary embodiment.

Each transducer element has a difference in time for an echo signal to arrive according to a location of a focal point. Accordingly, in order to generate ultrasound data, the ultrasound imaging apparatus needs to delay an echo signal arriving earlier at a transducer element to correspond to an echo signal arriving later at a transducer element.

Referring to FIG. 3, the time delay circuit includes a plurality of capacitors 320a through 320h, a plurality of input switches 310a through 310h for applying signals respectively to the corresponding capacitors 320a through 320h, and a plurality of output switches 330a through 330h for outputting signals respectively from the corresponding capacitors 320a through 320h. Although FIG. 3 shows the number of capacitors is 8, the number of capacitors is not limited thereto. In other words, the number of capacitors and the number of input/output switches included in the time delay circuit may be increased or decreased based on a maximum delay time to be applied.

Referring to FIG. 3, in the time delay circuit, an echo signal received from a corresponding transducer element may be applied as an input signal. The time delay circuit may store the applied echo signal in the capacitor 320a by controlling the input switch 310a connected to the capacitor 320a such that the input switch 310a is turned on. The time delay circuit may output, based on a set delay time, the echo signal stored in the capacitor 320a by controlling the output switch 330a connected to the capacitor 320a such that the output switch is turned on.

The time delay circuit may store received echo signals in the plurality of capacitors 320a through 320h by controlling the plurality of input switches 310a through 310h such that they are sequentially turned on. The time delay circuit may output, based on the set delay time, the echo signals stored in the plurality of capacitors 320a through 320h by controlling the plurality of output switches 330a through 330h respectively connected to the plurality of capacitors 320a through 320h such that the plurality of output switches 330a through 330h are sequentially turned on.

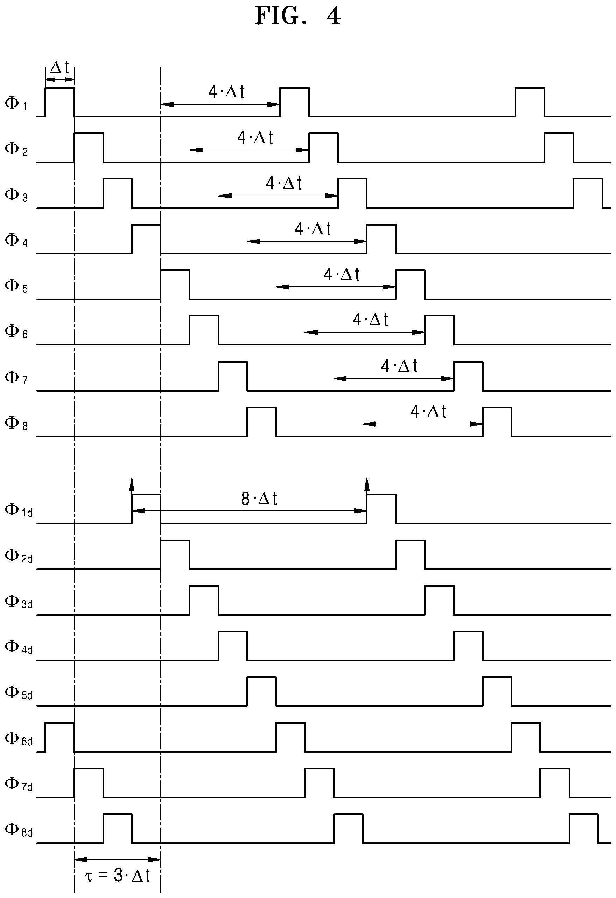

FIG. 4 illustrates signals used for a conventional ultrasound imaging apparatus to control an operation of a time delay circuit.

Referring to FIG. 4, in the conventional ultrasound imaging apparatus, input signals Φ1 through Φ8 respectively corresponding to the plurality of input switches 310a through 310h of the time delay circuit and output signals Φ1d through Φ8d respectively corresponding to the plurality of output switches 330a through 330h may be applied to the time delay circuit.

The conventional ultrasound imaging apparatus delays echo signals by using all capacitors included in the time delay circuit regardless of delay times set in the time delay circuit.

Referring to FIG. 4, in the time delay circuit, a delay time $\tau$ may be set to be three times a minimum delay time $\Delta t$. The time delay circuit of the conventional ultrasound imaging apparatus delays echo signals by using all eight capacitors regardless of the set delay times.

In detail, the conventional ultrasound imaging apparatus may apply an echo signal to the capacitor 320a by applying the input signal Φ1 for controlling the input switch 310a to be turned on at a first time. Furthermore, the conventional ultrasound imaging apparatus may output an echo signal stored in the capacitor 320a by applying the output signal Φ1d for controlling the output switch 330a to be turned on at a second time. In this case, the second time may be a time that occurs a preset delay time after the first time. That is, the second time may be a time that occurs three times the minimum delay time $\Delta t$ after the first time.

The conventional ultrasound imaging apparatus may also apply an echo signal to the capacitor 320a by applying the input signal Φ1 for controlling the input switch 310a to be turned on at a third time. The third time may be a time that occurs 8 times the minimum delay time $\Delta t$ after the first time. That is, the third time may be a time that occurs a time period after the first time, the time period corresponding to a result of multiplication of the number of capacitors included in the time delay circuit and the minimum delay time $\Delta t$.

The conventional ultrasound imaging apparatus may output an echo signal stored in the capacitor 320a by applying the output signal Φ1d for controlling the output switch 330a to be turned on at a fourth time. The fourth time may be a time that occurs 8 times the minimum delay time $\Delta t$ after the second time. In other words, the fourth time may be a time that occurs a time period after the second time, the time period corresponding to the result of multiplication of the number of capacitors included in the time delay circuit and the minimum delay time $\Delta t$.

When the output switches 330a through 330h are controlled to be turned on in response to the corresponding output signals Φ1d through Φ8d, output signals may be generated as noise. For example, clock harmonic leakage occurring at an output switch may be included as noise in an echo signal output from a capacitor.

In the time delay circuit of the conventional ultrasound imaging apparatus, noise included in an echo signal output from each capacitor may be generated repeatedly for each specific period. In detail, noise may be generated repeatedly for each period corresponding to a result of multiplication of the number of capacitors and the minimum delay time $\Delta t$. Referring to FIG. 4, noise may be generated repeatedly for every period corresponding to 8 times the minimum delay time $\Delta t$.

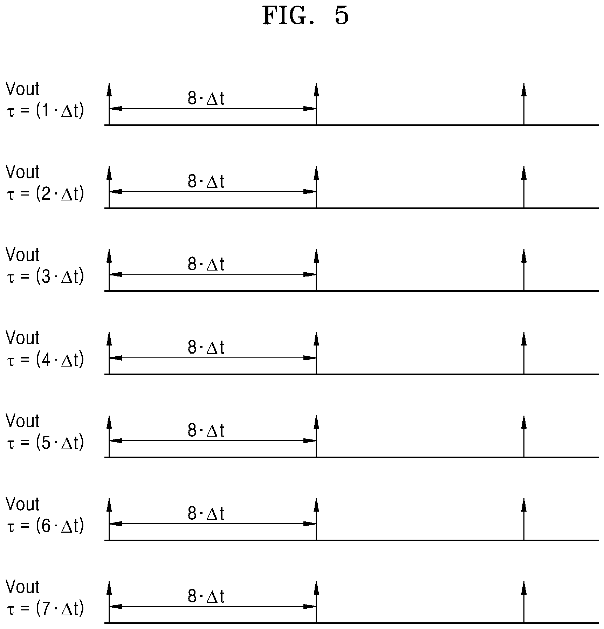

FIG. 5 illustrates noise generated according to a delay time in a time delay circuit of a conventional ultrasound imaging apparatus.

Referring to FIG. 5, noise generated in the time delay circuit of the conventional ultrasound imaging apparatus is the same regardless of a delay time set for each transducer element.

As described above with reference to FIG. 4, the time delay circuit of the conventional ultrasound imaging apparatus performs a time delay by using all capacitors regardless of a delay time set for each transducer element. Thus, each output switch corresponding to one of the capacitors included in the time delay circuit is controlled to be turned on such that noise may be repeatedly generated for each period corresponding to a result of multiplication of the number of capacitors and the minimum delay time $\Delta t$. Thus, noise in an echo signal output from the time delay circuit is repeatedly generated for each period corresponding to the result of multiplication of the number of capacitors and the minimum delay time $\Delta t$.

Furthermore, all the time delay circuits respectively connected to each of the transducer elements repeatedly output noise for each period corresponding to the result of multiplication of the number of capacitors and the minimum delay time $\Delta t$.

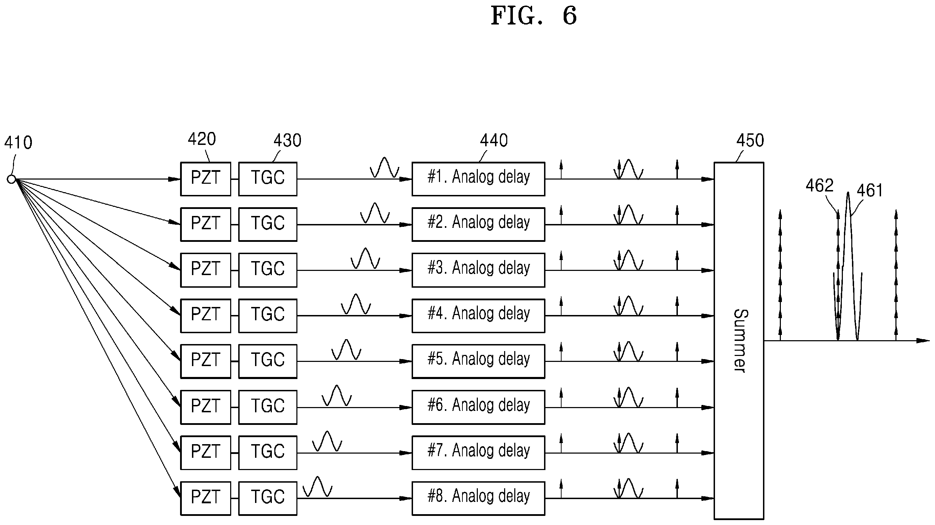

FIG. 6 illustrates a signal and noise generated when a conventional ultrasound imaging apparatus performs beamforming.

As described above, in the conventional ultrasound imaging apparatus, noise is repeatedly generated at predetermined time intervals regardless of a delay time $\tau$ set for each of the transducer elements.

Referring to FIG. 6, the conventional ultrasound imaging apparatus may transmit ultrasound signals to an object based on a location of a focus point 410 by using a plurality of transducer elements 420 and receive echo signals from the object. The conventional ultrasound imaging apparatus may perform, via compensators 430, time gain compensation or depth gain compensation of echo signals respectively received by the plurality of transducer elements 420. The conventional ultrasound imaging apparatus may delay the echo signals that have undergone the time gain compensation by using a plurality of time delay circuits 440 based on delay times respectively set for the plurality of transducer elements 420. The conventional ultrasound imaging apparatus may generate ultrasound data 461 by summing the delayed echo signals.

The conventional ultrasound imaging apparatus may delay an echo signal by using any of the time delay circuits of FIGS. 3 through 5. Thus, noise may be repeatedly generated at specific time intervals regardless of a delay time $\tau$ set for each of the transducer elements 420. When a summer 450 sums echo signals output from the plurality of time delay circuits 440, noise included in the echo signals may also be added thereto. Because noise is respectively generated in the plurality of time delay circuits 440 at the same time, noise included in the echo signals overlaps one another at a specific frequency. Thus, because the ultrasound data 461 generated by the conventional ultrasound imaging apparatus includes noise 462 overlapping at a specific frequency, an SNR is reduced, and an unclear ultrasound image is generated. Furthermore, because amplitudes and shapes of noise included in echo signals are not uniform, it is not easy to remove the noise 462 from the ultrasound data 461.

Therefore, beamforming needs to be performed so that noise does not overlap one another at a specific frequency.

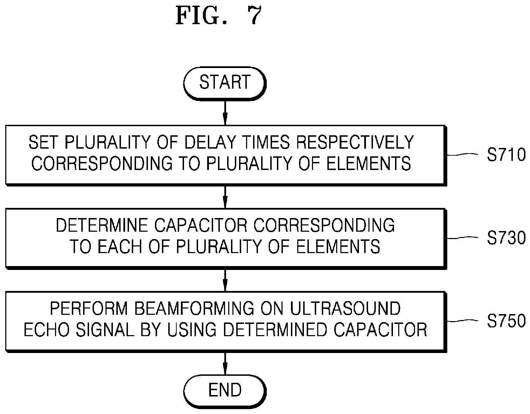

FIG. 7 is a flowchart of a method, performed by an ultrasound imaging apparatus, of performing beamforming, according to an exemplary embodiment.

Referring to FIG. 7, the ultrasound imaging apparatus may set a plurality of delay times respectively corresponding to a plurality of transducer elements (S710), determine a capacitor corresponding to each of the plurality of transducer elements (S730), and perform beamforming on an echo signal by using the determined capacitor (S750).

Referring to operation S710, the ultrasound imaging apparatus may set the delay times respectively corresponding to the transducer elements based on a location of a focal point. Different delay times may be respectively set for the transducer elements based on a depth of the focal point. For example, as the depth of the focal point decreases, a longer delay time may be set for a transducer element located closer to the focal point.

Referring to operation S730, the ultrasound imaging apparatus may determine a capacitor corresponding to a delay time of each of the transducer elements. In other words, the ultrasound imaging apparatus may determine a capacitor to be used for a time delay from among capacitors included in a time delay circuit connected to each of the transducer elements.

The ultrasound imaging apparatus may determine at least one capacitor to be used for the time delay from among the capacitors 320a through 320h included in the time delay circuit. The ultrasound imaging apparatus may determine the number of capacitors to be used for a time delay based on a set delay time.

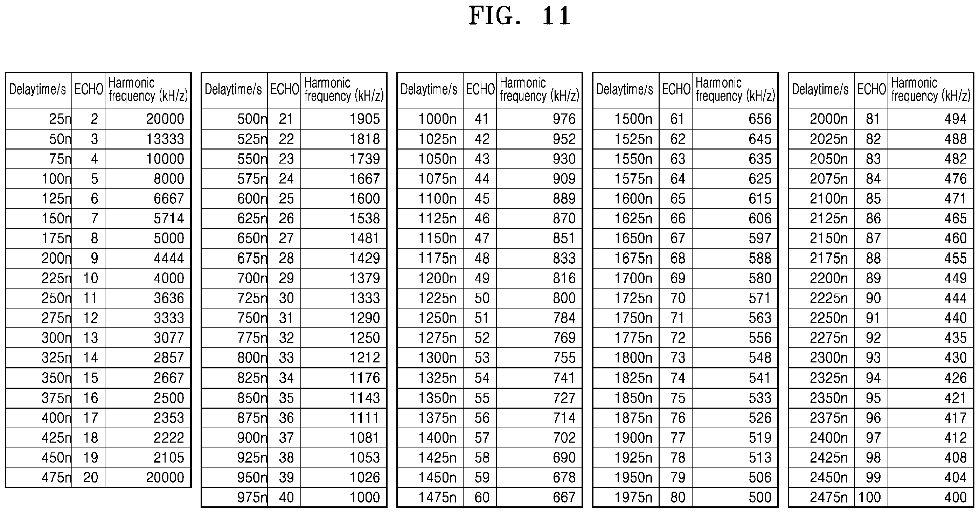

According to an exemplary embodiment, as shown in FIG. 11, the ultrasound imaging apparatus may determine the number of capacitors corresponding to each of the transducer elements by comparing a delay time set for each of the transducer elements with a preset reference time. In other words, the number of capacitors corresponding to each of the delay times may be preset, and the ultrasound imaging apparatus may determine the number of capacitors corresponding to the preset delay time as the number of capacitors to be used for a time delay. Details thereof will be described below with reference to FIG. 11.

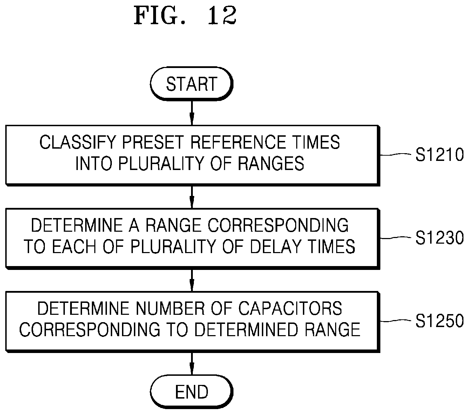

According to an exemplary embodiment, the ultrasound imaging apparatus may compare each of the delay times respectively set for the transducer elements with the preset reference time. The ultrasound imaging apparatus may determine a range corresponding to each of the delay times from among a plurality of ranges obtained via classification based on the preset reference time. The ultrasound imaging apparatus may determine the number of capacitors corresponding to the determined range as the number of capacitors to be used for a time delay. Details thereof will be described below with reference to FIG. 12.

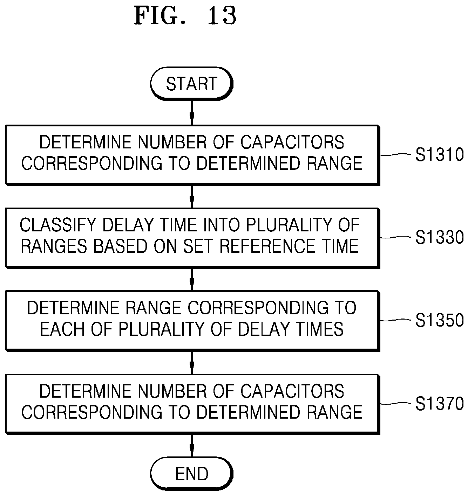

According to an exemplary embodiment, the ultrasound imaging apparatus may determine the number of capacitors corresponding to each of the transducer elements based on a longest delay time among the plurality of delay times respectively set for the transducer elements. Details thereof will be described below with reference to FIG. 13.

According to an exemplary embodiment, the ultrasound imaging apparatus may classify the longest delay time into a plurality of ranges based on the preset reference time and compare a delay time corresponding to each of the transducer elements with the preset referenced time, thereby determining a range corresponding to the delay time from among the plurality of ranges. The ultrasound imaging apparatus may determine the number of capacitors corresponding to the determined range. Details thereof will be described below with reference to FIG. 13.

According to an exemplary embodiment, the ultrasound imaging apparatus may classify the longest delay time into a plurality of ranges by applying a predetermined reference ratio to the longest delay time. Details thereof will be described below with reference to FIG. 13.

According to an exemplary embodiment, the ultrasound imaging apparatus may determine the number of capacitors corresponding to each of the transducer elements such that the number of transducer elements for which the same number of capacitors are set is less than or equal to a predetermined value. Details thereof will be described below with reference to FIG. 11.

According to an exemplary embodiment, the ultrasound imaging apparatus may determine the number of capacitors corresponding to each of the transducer elements, such that the number (e.g., 4) of types of a category (e.g., 2 capacitors, 3 capacitors, 10 capacitors, and 30 capacitors) indicating the number of capacitors set for each of the transducer elements is greater than or equal to a predetermined value. Details thereof will be described below with reference to FIG. 11.

Referring to operation S750, the ultrasound imaging apparatus may perform beamforming on a received echo signal by using the determined capacitor. The ultrasound imaging apparatus may perform time delays of echo signals respectively received from the transducer elements based on the delay times respectively for the transducer elements. The ultrasound imaging apparatus may generate ultrasound data by summing the delayed echo signals. The ultrasound imaging apparatus may generate an ultrasound image based on the ultrasound data and display the ultrasound image.

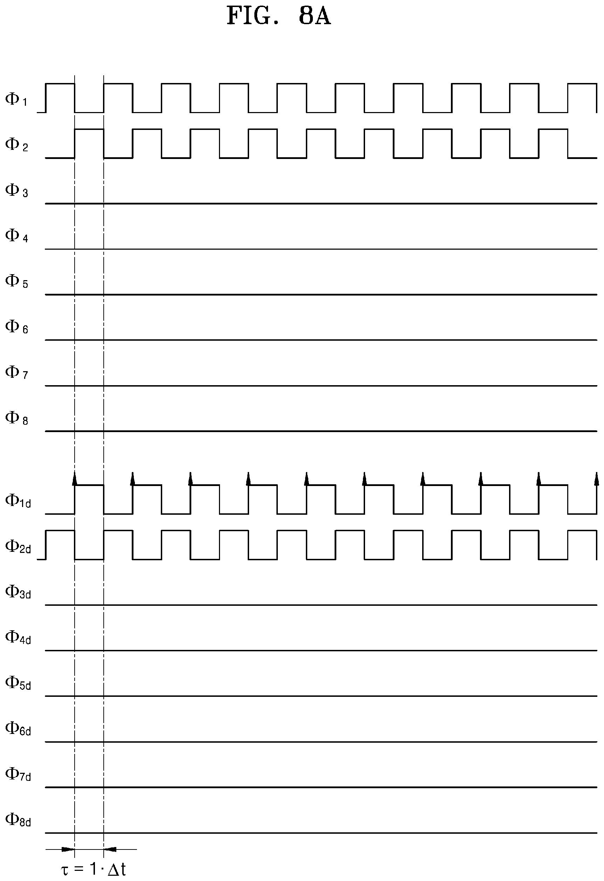

FIGS. 8A through 8G respectively show that an ultrasound imaging apparatus perform time delays by using capacitors, according to an exemplary embodiment.

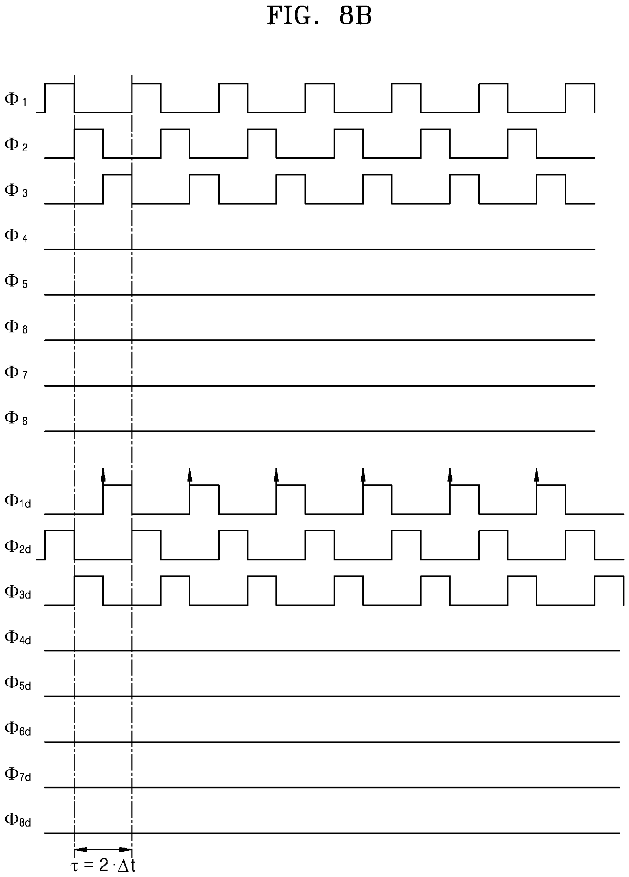

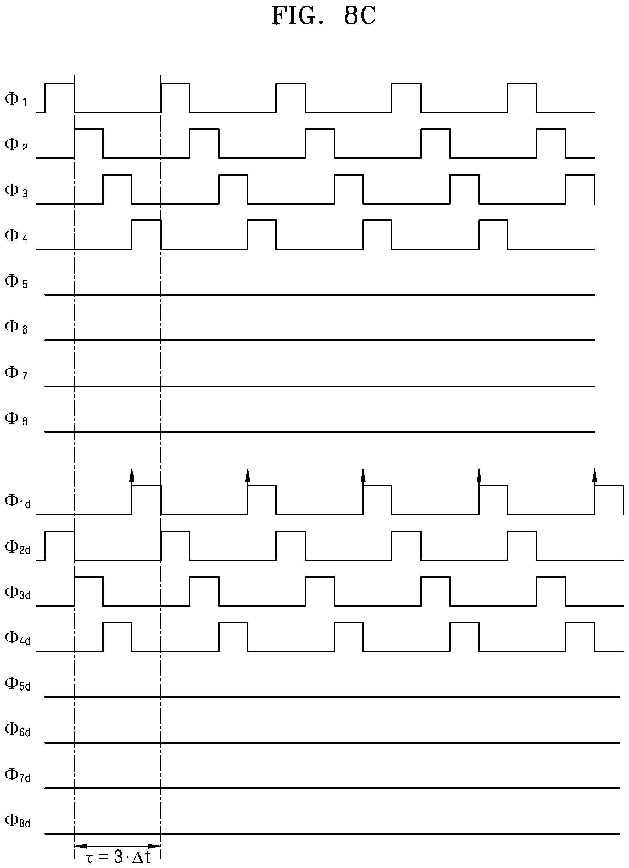

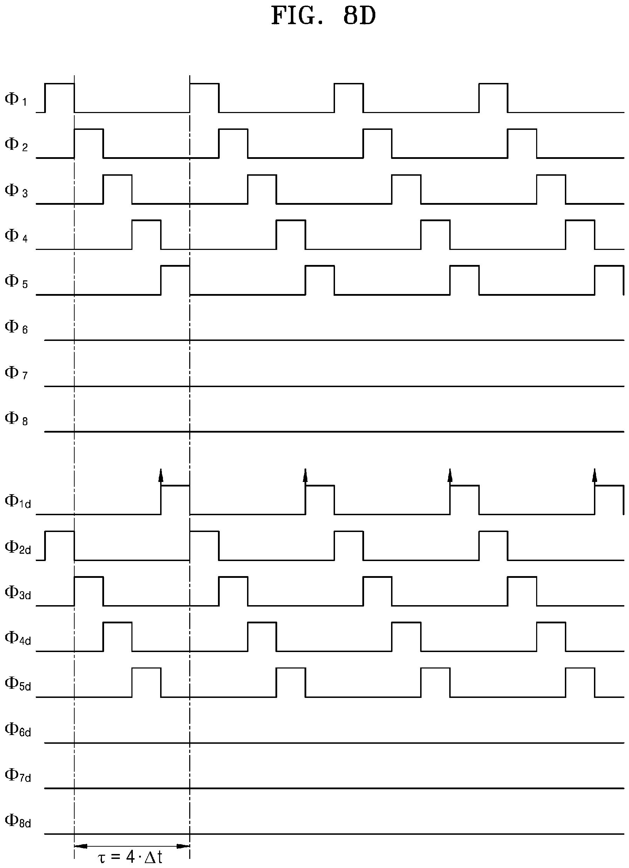

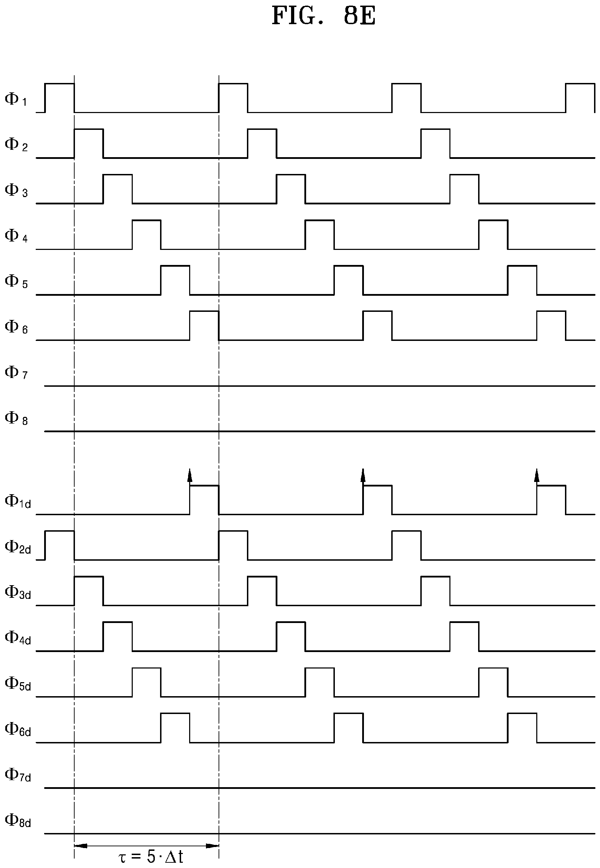

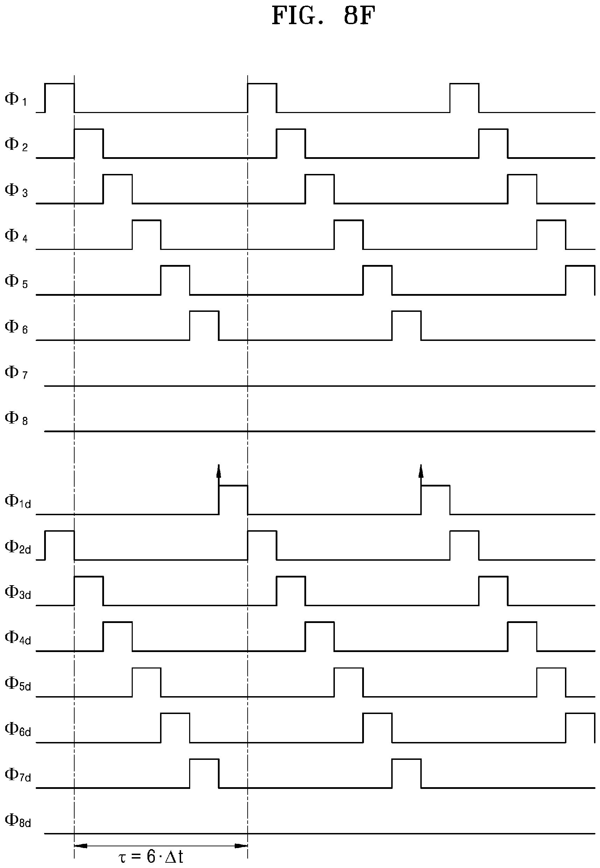

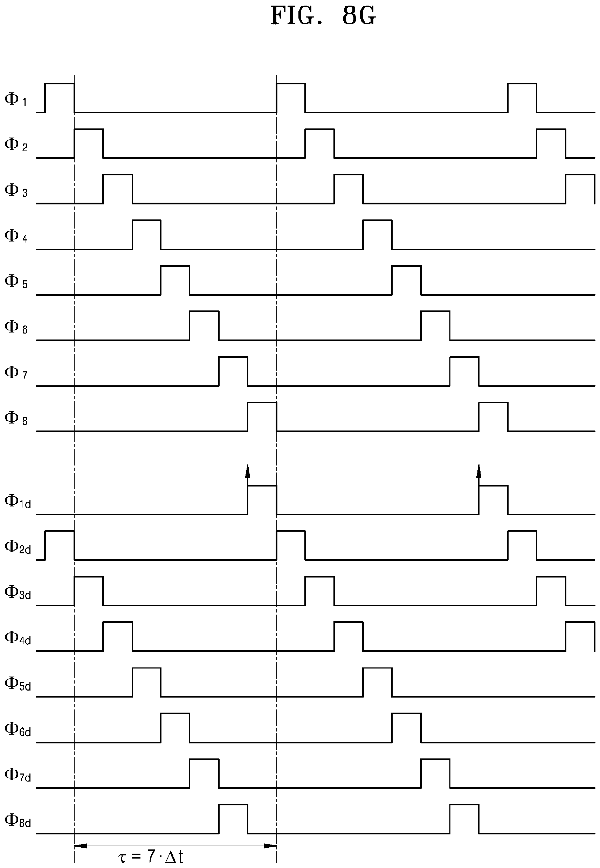

In detail, FIG. 8A shows that the ultrasound imaging apparatus performs a time delay by using two capacitors, FIG. 8B shows that the ultrasound imaging apparatus performs a time delay by using three capacitors, FIG. 8C shows that the ultrasound imaging apparatus performs a time delay by using four capacitors, FIG. 8D shows that the ultrasound imaging apparatus performs a time delay by using five capacitors, FIG. 8E shows that the ultrasound imaging apparatus performs a time delay uses six capacitors, FIG. 8F shows that the ultrasound imaging apparatus performs a time delay by using seven capacitors, and FIG. 8G shows that the ultrasound imaging apparatus performs a time delay by using eight capacitors.

The ultrasound imaging apparatus may determine, based on a delay time set for a transducer element, the number of capacitors to be used for a time delay of an echo signal received by the transducer element. Furthermore, the ultrasound imaging apparatus may perform the time delay of the echo signal based on the determined number of capacitors.

Referring to FIG. 8A, when a delay time $\tau$ set for a transducer element is a minimum delay time $\Delta t$, the ultrasound imaging apparatus may select two capacitors as capacitors to be used for the time delay.

Referring to FIG. 8B, when the delay time $\tau$ set for the transducer element is twice the minimum delay time $\Delta t$, the ultrasound imaging apparatus may select three capacitors as capacitors to be used for the time delay.

Referring to FIG. 8C, when the delay time $\tau$ set for the transducer element is three times the minimum

delay time $\Delta t$, the ultrasound imaging apparatus may select four capacitors as capacitors to be used for the time delay.

Referring to FIG. 8D, when the delay time $\tau$ set for the transducer element is four times the minimum delay time $\Delta t$, the ultrasound imaging apparatus may select five capacitors as capacitors to be used for the time delay.

Referring to FIG. 8E, when the delay time $\tau$ set for the transducer element is 5 times the minimum delay time $\Delta t$, the ultrasound imaging apparatus may select six capacitors as capacitors to be used for the time delay.

Referring to FIG. 8F, when the delay time $\tau$ set for the transducer element is 6 times the minimum delay time $\Delta t$, the ultrasound imaging apparatus may select seven capacitors as capacitors to be used for the time delay.

Referring to FIG. 8G, when the delay time $\tau$ set for the transducer element is seven times the minimum delay time $\Delta t$, the ultrasound imaging apparatus may select eight capacitors as capacitors to be used for the time delay.

That is, referring to FIGS. 8A through 8G, a relationship among the delay time $\tau$, the minimum delay time $\Delta t$, and the number of capacitors used for a time delay is defined by using Equation 1.

【Equation 1】

$$\tau = \Delta t \times (N-1)$$

where $\tau$ is a delay time, $\Delta t$ is a minimum delay time, and N is the number of capacitors used for a time delay.

Moreover, a result of comparing time delays performed by a conventional ultrasound imaging apparatus and an ultrasound imaging apparatus according to an exemplary embodiment in which the delay time $\tau$ is equally set to three times the minimum delay time $\Delta t$ is as follows.

Referring to FIG. 4, the conventional ultrasound imaging apparatus performs a time delay by using all capacitors regardless of a delay time set for a transducer element. In other words, since the conventional ultrasound imaging apparatus performs a time delay using all capacitors, a signal is not stored in a capacitor for a time that is four times the minimum delay time $\Delta t$.

Referring to FIG. 8C, the ultrasound imaging apparatus according to the exemplary embodiment determines the number of capacitors according to a delay time set for a transducer element. Thus, unlike the conventional ultrasound imaging apparatus, the ultrasound imaging apparatus according to the exemplary embodiment may adjust the number of capacitors such that a time when a signal is not stored in a capacitor used for a time delay is not uniform.

The ultrasound imaging apparatus according to the exemplary embodiment may determine the number of capacitors such that there is no time during which a signal is not stored in a capacitor used for a time delay. The ultrasound imaging apparatus according to the invention determines the number of capacitors such that a time when a signal is not stored in a capacitor used for a time delay is less than a predetermined time.

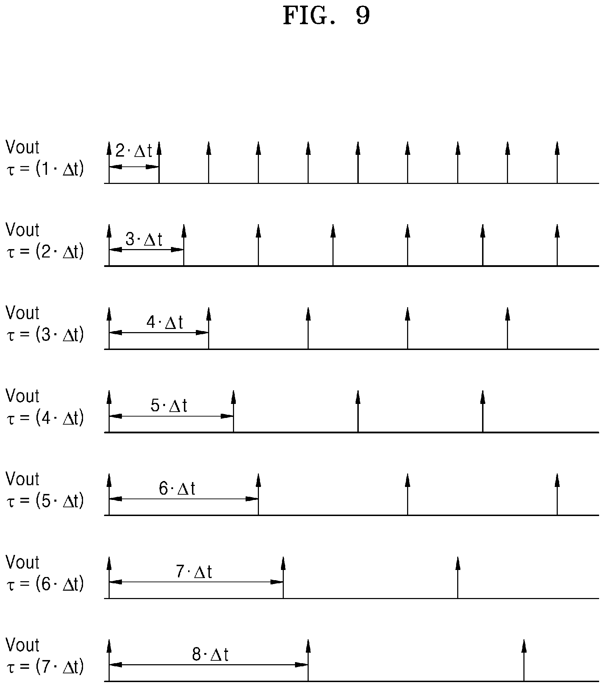

FIG. 9 illustrates noise generated according to a delay time in a time delay circuit according to an exemplary embodiment.

Referring to FIG. 8A, the ultrasound imaging apparatus may perform a time delay by using capacitors having a number determined as two. Accordingly, noise may be generated for each period corresponding to twice the minimum delay time $\Delta t$.

Referring to FIG. 8B, the ultrasound imaging apparatus may perform a time delay by using capacitors having a number determined as three. Accordingly, noise may be generated for each period corresponding to three times the minimum delay time $\Delta t$.

Referring to FIG. 8C, the ultrasound imaging apparatus may perform a time delay by using capacitors having a number determined as four. Thus, noise may be generated for each period corresponding to four times the minimum delay time $\Delta t$.

Referring to FIG. 8D, the ultrasound imaging apparatus may perform a time delay by using capacitors having a number determined as five. Thus, noise may be generated for each period corresponding to five times the minimum delay time $\Delta t$.

Referring to FIG. 8E, the ultrasound imaging apparatus may perform a time delay by using capacitors having a number determined as six. Accordingly, noise may be generated for each period corresponding to six times the minimum delay time $\Delta t$.

Referring to FIG. 8F, the ultrasound imaging apparatus may perform a time delay by using capacitors having a number determined as seven. Thus, noise may be generated for each period corresponding to seven times the minimum delay time $\Delta t$.

Referring to FIG. 8G, the ultrasound imaging apparatus may perform a time delay by using capacitors having a number determined as eight. Thus, noise may be generated for each period corresponding to eight times the minimum delay time $\Delta t$.

Referring to FIGS. 8A through 8G, noise included in an echo signal is output from the time delay circuit as shown in FIG. 9.

Referring to FIG. 9, noise included in an echo signal output from the time delay circuit may occur for a different period depending on the number of capacitors used for a time delay. In other words, noise included in the echo signal may be generated at a different frequency depending on the number of capacitors used for the time delay.

Referring to FIG. 9, a frequency of noise included in an echo signal output from the time delay circuit may have a relationship defined by Equation 2.

【Equation 2】

$$f(\text{Hz}) = \frac{1}{\tau + \Delta t}$$

where $\tau$ is a delay time, $\Delta t$ is a minimum delay time, and $f$ is the frequency of the generated noise.

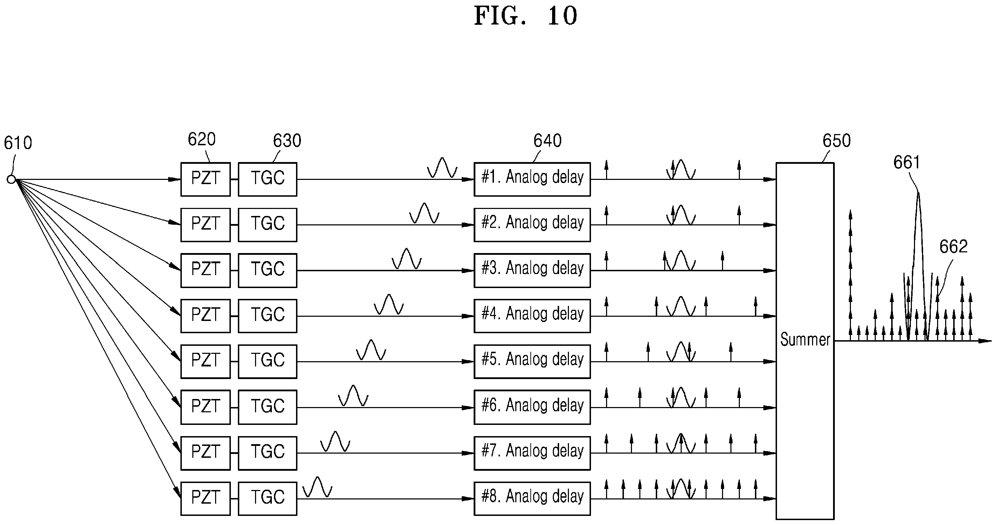

FIG. 10 illustrates a signal and noise generated when an ultrasound imaging apparatus performs beamforming, according to an exemplary embodiment

Referring to FIG. 10, the ultrasound imaging apparatus may transmit ultrasound signals to an object by using a plurality of transducer elements 620 and receive echo signals from the object. The ultrasound imaging apparatus may perform, via compensators 630, time gain compensation or depth gain compensation of echo signals respectively received by the plurality of transducer elements 620. The ultrasound imaging apparatus may delay the echo signals that have undergone the time gain compensation by using a plurality of time delay circuits 640 based on delay times respectively set for the plurality of transducer elements 620. The ultrasound imaging apparatus may generate ultrasound data 661 by summing the delayed echo signals via a summer 650.

When the summer 650 sums the echo signals output from the plurality of time delay circuits 640, noise included in the echo signals may also be added thereto. Noise respectively generated by the plurality of time delay circuits 650 may have different frequencies. Noise 662 in the ultrasound data 661 may be generated at various frequencies and dispersed without overlapping at a specific frequency.

An amplitude of the noise 662 in the ultrasound data 661 generated by the ultrasound imaging apparatus according to the exemplary embodiment is smaller than the amplitude of the noise 462 in the ultrasound data 461 generated by the conventional ultrasound imaging apparatus. Accordingly, the ultrasound imaging apparatus of the exemplary embodiment may acquire ultrasound data having an improved SNR and generate a clearer ultrasound image, as compared to the conventional ultrasound imaging apparatus.

FIG. 11 illustrates a correlation among a delay time, noise, and the number of capacitors used when an ultrasound imaging apparatus performs beamforming, according to an exemplary embodiment.

In detail, FIG. 11 is a table showing the number of capacitors and a frequency of noise corresponding to a reference time when a minimum delay time $\Delta t$ is 25 ns.

Referring to FIG. 11, as the delay time increases, the number of capacitors used for a time delay increases, and noise with a low- frequency component occurs.

In a conventional ultrasound imaging apparatus, because a time delay is performed by using all capacitors regardless of a delay time set for a transducer element, noise with low-frequency components overlaps one another so that an SNR of ultrasound data is reduced. However, in an ultrasound imaging apparatus according to an exemplary embodiment disclosed herein, because a time delay is performed using a capacitor determined based on a delay time set for a transducer element, noise with a high frequency component may be generated. Accordingly, the ultrasound imaging apparatus according to the exemplary embodiment may prevent overlap of noise included in ultrasound data and disperse the noise.

According to an exemplary embodiment, the ultrasound imaging apparatus may determine the number of capacitors corresponding to each of a plurality of transducer elements by comparing a delay time set for each of the transducer elements with a preset reference time. For example, as shown in FIG. 11, the ultrasound imaging apparatus may determine the number of capacitors corresponding to each preset reference time as the number of capacitors corresponding to a transducer element. The ultrasound imaging apparatus may determine the number of capacitors corresponding to a delay time $\tau$ set for each of the transducer elements by comparing the delay time $\tau$ with a preset reference time. In detail, when the minimum delay time $\Delta t$ is 25 ns and set delay times ($\tau$) are 100 ns and 225 ns, the ultrasound imaging apparatus may determine the number of capacitors corresponding to the delay time of 100 ns as 5 and the delay time of 225 ns as 10.

According to an exemplary embodiment, the ultrasound imaging apparatus may determine the number of capacitors used for a time delay so that noise included in echo signals output from time delay circuits does not overlap one another.

For example, the ultrasound imaging apparatus may increase the number of capacitors corresponding to one of the transducer elements having the same number of capacitors used for a time delay. In detail, the ultrasound imaging apparatus may determine the number of capacitors as n based on a first delay time corresponding to a first transducer element. Furthermore, the ultrasound imaging apparatus may determine the number of capacitors as n based on a second delay time corresponding to a second transducer element. In this case, noise included in an echo signal output from a first time delay circuit connected to the first transducer element may overlap with noise included in an echo signal output from a second time delay circuit connected to the second transducer element. The ultrasound imaging apparatus may determine the number of capacitors corresponding to the second transducer element as n+1 such that the noise may not overlap each another.

As another example, the ultrasound imaging apparatus may determine the number of capacitors corresponding to each of the transducer elements such that the number of transducer elements delaying a time by using the same number of capacitors is less than or equal to a predetermined value. In detail, the ultrasound imaging apparatus may determine the number of capacitors corresponding to each of the transducer elements such that the number of transducer elements having the same number of capacitors used for a time delay is less than or equal to one third of a total number of transducer elements. The ultrasound imaging apparatus may increase the number of capacitors corresponding to one of the transducer elements having the same number of capacitors used for a time delay.

As another example, the ultrasound imaging apparatus may determine the number of capacitors used for a time delay such that an SNR of ultrasound data at frequencies where a noise level is maximum is less than or equal to a predetermined value. In detail, the ultrasound imaging apparatus may determine the number of capacitors corresponding to each of the transducer elements such that an SNR of ultrasound data at a frequency where a noise level is highest is greater than or equal to 45 dB. The ultrasound imaging apparatus may determine to increase the number of capacitors corresponding to one of the transducer elements having the same number of capacitors used for a time delay.

As another example, the ultrasound imaging apparatus may determine the number of capacitors so that the number of capacitors corresponding to each of the transducer elements may be of various types. In other words, the ultrasound imaging apparatus may determine the number of capacitors corresponding to each of the transducer elements such that the number of categories indicating the number of capacitors corresponding to each of the transducer elements is greater than or equal to a predetermined value.

According to an exemplary embodiment, the ultrasound imaging apparatus may determine the number of capacitors used for a time delay, which correspond to each of the transducer element, and classify the determined number of capacitors into categories as shown in FIG. 11. A category may indicate the number of capacitors set for each of the transducer elements.

For example, the ultrasound imaging apparatus may determine a type of a category indicating the number of capacitors that are to be used for a time delay. In detail, when the number of capacitors available for use in the time delay is 8, the ultrasound imaging apparatus may determine a category to have 8 types of 1, 2, 3, 4, 5, 6, 7, and 8. Referring to FIG. 11, because the number of capacitors available for use in the time delay is 100, the ultrasound imaging apparatus may determine a category to have 100 types of 1 through 100.

As another example, the ultrasound imaging apparatus may determine a category to correspond to the number of capacitors used for a time delay, which correspond to each of the transducer elements. In detail, when the numbers of capacitors used for the time delay are 3, 6, 7, and 8, the ultrasound imaging apparatus may determine the category to have 4 types of 3, 6, 7, and 8.

According to an exemplary embodiment, the ultrasound imaging apparatus may determine the number of capacitors corresponding to each of the transducer elements such that the number of types of a category is greater than or equal to a predetermined value. For example, when the number of capacitors available for use in a time delay is 8, the ultrasound imaging apparatus may determine the number of capacitors such that the number of types of a category is greater than or equal to 5. In detail, the ultrasound imaging apparatus may determine the number of capacitors corresponding to each of the transducer elements as corresponding to a category indicating that the numbers of capacitors to be used for the time delay are 2, 4, 5, 7, 8. The ultrasound imaging apparatus may determine again the number of capacitors to use five capacitors for a transducer element for which the number of capacitors used for a time delay was determined as four.

FIG. 12 is a flowchart of a method, performed by an ultrasound imaging apparatus, of performing beamforming, according to an exemplary embodiment.

Referring to FIG. 12, the ultrasound imaging apparatus may classify a delay time into a plurality of ranges based on a preset reference time (S1210), determine a range corresponding to a delay time set for each of a plurality of transducer elements from among the plurality of ranges (S1230), and determine the number of capacitors corresponding to the determined range as the number of capacitors corresponding to the transducer element (S1250).

Referring to operation S1210, the ultrasound imaging apparatus may classify the delay time into the plurality of ranges based on the preset reference time for the transducer elements. For example, as shown in FIG. 11, the ultrasound imaging apparatus may classify the delay time $\tau$ into a plurality of ranges based on a preset reference time. In detail, the ultrasound imaging apparatus may classify the delay time into a first range from 0 ns until 25 ns, a second range from 25 ns until 50 ns, a third range from 50 ns until 75 ns, and a fourth range from 75 ns until 100 ns.

Referring to operation S1230, by comparing a delay time set for each of the transducer elements with the preset reference time, the ultrasound imaging apparatus may determine a range corresponding to the delay time set for the transducer element from among the plurality of ranges obtained via classification based on the preset reference time. For example, when a set delay time $\tau$ is 90 ns, the ultrasound imaging apparatus may determine the fourth range as a range corresponding to the set delay time $\tau$ by comparing 90 ns that is the set delay time $\tau$ with each of 25ns, 50ns, 75ns, and 100ns that are preset reference times.

Referring to operation S1250, the ultrasound imaging apparatus may determine the number of capacitors corresponding to the determined range as the number of capacitors corresponding to the transducer element. For example, when the ultrasound imaging apparatus determines the fourth range as a range corresponding to the delay time $\tau$, the ultrasound imaging

apparatus may determine 5, which is the number of capacitors corresponding to the fourth range, as the number of capacitors corresponding to the transducer element. In other words, the ultrasound imaging apparatus may perform a time delay on an echo signal received by the transducer element by using five capacitors.

FIG. 13 is a flowchart of a method, performed by an ultrasound imaging apparatus, of performing beamforming, according to an exemplary embodiment.

Referring to FIG. 13, the ultrasound imaging apparatus may set a reference time by applying a reference ratio to a longest delay time among a plurality of delay times respectively set for transducer elements (S1310), classify the longest delay time into a plurality of ranges based on the set reference time (S1330), determine a range corresponding to a delay time set for each of the transducer elements from among the plurality of ranges (S1350), and determine the number of capacitors corresponding to the determined range as the number of capacitors corresponding to the transducer element (S1370).

Referring to operation S1310, the ultrasound imaging apparatus may set the reference time by applying the predetermined reference ratio to the longest delay time. For example, the ultrasound imaging apparatus may set the reference time by applying a multiple of a minimum delay time $\Delta t$ to the longest delay time as a reference ratio. In detail, the ultrasound imaging apparatus may set times that are multiples of the minimum delay time $\Delta t$ as the reference time by applying a multiple of the minimum delay time $\Delta t$ to the longest delay time as the reference ratio. As another example, the ultrasound imaging apparatus may set the reference time by applying the longest delay time to a logarithmic function. In detail, the ultrasound imaging apparatus may set a value obtained by multiplying the longest delay time by log(n) (n is a real number in a range from 1 to 10) as the reference time. As another example, the ultrasound imaging apparatus may set, as the reference time, a multiple of a value obtained by dividing the longest delay time by the number of capacitors that are to be used for a time delay. In detail, when the longest delay time is 1000 ns and the number of capacitors available for use in the time delay is 100, the ultrasound imaging apparatus may determine the reference time to be a multiple of 10 ns. In other words, the ultrasound imaging apparatus may determine the reference time to be 10ns, 20ns, 30ns, ..., and 1000ns.

Referring to operation S1330, the ultrasound imaging apparatus may classify the delay time into the plurality of ranges based on the reference time set in operation S1310. For example, when the reference time is determined to be a multiple of 10 ns in operation S1310, the ultrasound imaging apparatus may classify the delay time into a first range from 0 ns until 10 ns, a second range from 10 ns until 20 ns, a third range from 20 ns until 30 ns, and a fourth range from 30 ns until 40 ns.

Referring to operation S1350, by comparing a delay time set for each of the transducer elements with the reference time set in operation S 1330, the ultrasound imaging apparatus may determine a range corresponding to the delay time. For example, when a set delay time $\tau$ is 25 ns, the ultrasound imaging apparatus may determine the third range as a range corresponding to the set delay time $\tau$ by comparing 25 ns that is the set delay time $\tau$ with each of 10ns, 20ns, 30ns, and 40ns that are preset reference times.

Referring to operation S1370, the ultrasound imaging apparatus may determine the number of capacitors corresponding to the determined range as the number of capacitors corresponding to the transducer element. For example, when the ultrasound imaging apparatus determines the third range as the range corresponding to the delay time $\tau$, the ultrasound imaging apparatus may determine 4 that is the number of capacitors corresponding to the third range as the number of capacitors corresponding to the transducer element. In other words, the ultrasound imaging apparatus may perform a time delay on an echo signal received by the transducer element by using four capacitors.

Moreover, the ultrasound imaging apparatus according to the disclosed exemplary embodiments may flexibly determine, based on a delay time set for each of the transducer elements, the number of capacitors used for performing a time delay of an echo signal received from the transducer element. Thus, the ultrasound imaging apparatus may prevent overlap of noise included in echo signals by adjusting the number of capacitors corresponding to each of the transducer elements. Accordingly, an SNR of ultrasound data generated by the ultrasound imaging apparatus may be improved.

The disclosed exemplary embodiments may be implemented through computer-readable recording media having stored thereon computer-executable instructions and data. The instructions may be stored in the form of program code, and when executed by a processor, generate a predefined program module to perform a preset operation. Furthermore, when executed by the processor, the instructions may perform predetermined operations according to the exemplary embodiments.

The disclosed exemplary embodiments have been described above with reference to the accompanying drawings. It will be understood by those of ordinary skill in the art that various changes in form and details may be made therein as far as they fall under the scope of the appended claims. The disclosed exemplary embodiments are examples only and are not to be construed for purposes of limitation.

## Claims

1. A method of generating an ultrasound image, the method comprising:

setting a focal point in an object (10);
transmitting, based on a location of the focal point (410), ultrasound signals into the object (10) by using a plurality of elements in a transducer (420);
receiving, via the plurality of elements (420), ultrasound echo signals obtained by reflecting the ultrasound signals from the object (10); and
performing beamforming on the received ultrasound echo signals,
wherein the performing beamforming of the received ultrasound echo signals comprises:

setting (S710), based on the location of the focal point (410), a plurality of delay times respectively corresponding to the plurality of elements;
determining (S730) the number of capacitors (320) to be used for time delay, respectively corresponding to the plurality of elements based on the set plurality of delay times, such that a time when a signal is not stored in a capacitor (320) used for the time delay is less than a predetermined time; and
respectively performing (S750) the beamforming on the received ultrasound echo signals by using the determined number of capacitors (320).

2. The method of claim 1, wherein the number of capacitors (320) corresponding to each of the plurality of elements is determined by comparing each of the set plurality of delay times with a preset reference time (S1210).

3. The method of claim 2, wherein the determining of the number of capacitors (320) comprises:

determining (S1230) a range corresponding to each of the set plurality of delay times from among a plurality of ranges obtained via classification based on the preset reference time by comparing each of the set plurality of delay times with the preset reference time; and
determining (S1250) the number of capacitors (320) corresponding to the determined range.

4. The method of claim 1, wherein the number of capacitors (320) corresponding each of the plurality of elements is determined by comparing a longest delay time among the set plurality of delay times with each of the set plurality of delay times.

5. The method of claim 1,
wherein the number of a plurality of elements having the same determined number of capacitors (320) is less than or equal to a predetermined value.

6.  The method of claim 1,
    wherein the number of types of a category indicating the determined number of capacitors (320) is greater than or equal to a predetermined value.

7.  The method of claim 1, further comprising:

    converting the beamformed ultrasound echo signals into ultrasound image signals; and
    displaying the ultrasound image signals.

8.  A computer program product comprising a computer-readable storage medium (150) including instructions, which when executed by a processor (120) of an ultrasound imaging apparatus (100) cause the processor (120) to:

    set a focal point in an object (10);
    transmit, based on a location of the focal point (410), ultrasound signals into the object (10) by using a plurality of elements in a transducer (420);
    receive, via the plurality of elements, ultrasound echo signals obtained by reflecting the ultrasound signals from the object (10); and
    perform beamforming on the received ultrasound echo signals,
    wherein the performing beamforming of the received ultrasound echo signals comprises:

        setting (S710), based on the location of the focal point (410), a plurality of delay times respectively corresponding to the plurality of elements;
        determining (S730) the number of capacitors (320) to be used for time delay, respectively corresponding to the plurality of elements based on the set plurality of delay times, such that a time when a signal is not stored in a capacitor (320) used for the time delay is less than a predetermined time; and
        respectively performing (S750) the beamforming on the received ultrasound echo signals by using the determined number of capacitors (320).

9.  An ultrasound imaging apparatus (100) comprising:

    a processor (120) configured to set a focal point in an object (10);
    a transducer (110) including a plurality of elements and configured to transmit ultrasound signals into the object (10) based on a location of the focal point (410) and receive ultrasound echo signals obtained by reflecting the ultrasound signals from the object (10); and
    a plurality for capacitors (420) used to perform beamforming on the received ultrasound echo signals,
    wherein the processor (120) is configured to:

        set (S710), based on the location of the focal point (410), a plurality of delay times respectively corresponding to the plurality of elements;
        determine (S730) the number of capacitors (320) to be used for time delay, respectively corresponding to the plurality of elements based on the set plurality of delay times, such that a time when a signal is not stored in a capacitor (320) used for the time delay is less than a predetermined time; and
        respectively perform (S750) the beamforming on the received ultrasound echo signals by using the determined number of capacitors (320).

10. The ultrasound imaging apparatus (100) of claim 9, wherein the processor (120) is further configured to determine the number of capacitors (320) corresponding to each of the plurality of elements by comparing each of the set plurality of delay times with a preset reference time (S1210).

11. The ultrasound imaging apparatus (100) of claim 10, wherein the processor (120) is further configured to:

    determine (S1230) a range corresponding to each of the set plurality of delay times from among a plurality of ranges obtained via classification based on the preset reference time by comparing each of the set plurality of delay times with the preset reference time; and
    determine (S1250) the number of capacitors (320) corresponding to the determined range.

12. The ultrasound imaging apparatus (100) of claim 9, wherein the processor (120) is further configured to determine the number of capacitors (320) corresponding each of the plurality of elements by comparing a longest delay time among the set plurality of delay times with each of the set plurality of delay times.

13. The ultrasound imaging apparatus (100) of claim 9, wherein the number of a plurality of elements having the same determined number of capacitors (320) is less than or equal to a predetermined value.

14. The ultrasound imaging apparatus (100) of claim 9, wherein the number of types of a category indicating the determined number of capacitors is greater than or equal to a predetermined value.

15. The ultrasound imaging apparatus (100) of claim 9, wherein the processor (120) is further configured to convert the beamformed ultrasound echo signals into ultrasound image signals,
the ultrasound imaging apparatus (100) further comprising a display (140) displaying the ultrasound image signals.

## Patentansprüche

1. Verfahren zur Erzeugung eines Ultraschallbildes, wobei das Verfahren Folgendes umfasst:

Einstellen eines Brennpunkts in einem Objekt (10);
Übertragen von Ultraschallsignalen in das Objekt (10), basierend auf einem Standort des Brennpunkts (410) und unter Verwendung einer Vielzahl von Elementen in einem Wandler (420);
Empfangen, über die Vielzahl von Elementen (420), von Ultraschallechosignalen, die durch Reflektieren der Ultraschallsignale von dem Objekt (10) erhalten werden; und
Durchführen einer Strahlformung an den empfangenen Ultraschallechosignalen,
wobei das Durchführen der Strahlformung der empfangenen Ultraschallechosignale Folgendes umfasst:

Einstellen (S710), basierend auf dem Standort des Brennpunkts (410), einer Vielzahl von Verzögerungszeiten, die jeweils der Vielzahl von Elementen entsprechen;
Bestimmen (S730) der Anzahl von für die Zeitverzögerung zu verwendenden Kondensatoren (320), die jeweils der Vielzahl von Elementen entsprechen, basierend auf der eingestellten Vielzahl von Verzögerungszeiten, so dass eine Zeit, in der ein Signal nicht in einem für die Zeitverzögerung verwendeten Kondensator (320) gespeichert ist, kleiner als eine vorbestimmte Zeit ist; und
jeweiliges Durchführen (S750) der Strahlformung an den empfangenen Ultraschallechosignalen unter Verwendung der bestimmten Anzahl von Kondensatoren (320).

2. Verfahren nach Anspruch 1, wobei die Anzahl der Kondensatoren (320), die jedem aus der Vielzahl von Elementen entspricht, durch Vergleichen jeder aus der eingestellten Vielzahl von Verzögerungszeiten mit einer voreingestellten Referenzzeit bestimmt wird (S1210).

3. Verfahren nach Anspruch 2, wobei das Bestimmen der Anzahl der Kondensatoren (320) Folgendes umfasst:

Bestimmen (S1230) eines Bereichs, der jeder aus der eingestellten Vielzahl von Verzögerungszeiten entspricht, aus einer Vielzahl von Bereichen, die durch Klassifizierung basierend auf der voreingestellten Referenzzeit erhalten werden, durch Vergleichen jeder aus der eingestellten Vielzahl von Verzögerungszeiten mit der voreingestellten Referenzzeit; und
Bestimmen (S1250) der Anzahl der Kondensatoren (320), die dem bestimmten Bereich entsprechen.

4. Verfahren nach Anspruch 1, wobei die Anzahl der Kondensatoren (320), die jedem aus der Vielzahl von Elementen entspricht, bestimmt wird, indem eine längste Verzögerungszeit aus der eingestellten Vielzahl von Verzögerungszeiten mit jeder aus der eingestellten Vielzahl von Verzögerungszeiten verglichen wird.

5. Verfahren nach Anspruch 1, wobei die Anzahl einer Vielzahl von Elementen, die die gleiche bestimmte Anzahl von Kondensatoren (320) aufweist, kleiner oder gleich einem vorbestimmten Wert ist.

6. Verfahren nach Anspruch 1, wobei die Anzahl der Arten einer Kategorie, die die bestimmte Anzahl von Kondensatoren (320) angibt, größer oder gleich einem vorbestimmten Wert ist.

7. Verfahren nach Anspruch 1, das ferner Folgendes umfasst:

Umwandeln der strahlgeformten Ultraschallechosignale in Ultraschallbildsignale; und
Anzeigen der Ultraschallbildsignale.

8. Computerprogrammprodukt, das ein computerlesbares Speichermedium (150) umfasst, das Anweisungen enthält, die, wenn sie von einem Prozessor (120) einer Ultraschallbildgebungsvorrichtung (100) ausgeführt werden, den Prozessor (120) zu Folgendem veranlassen:

Einstellen eines Brennpunkts in einem Objekt (10);
Übertragen von Ultraschallsignalen in das Objekt (10) basierend auf einem Standort des Brennpunkts (410) und unter Verwendung einer Vielzahl von Elementen in einem Wandler (420);
Empfangen, über die Vielzahl von Elementen, von Ultraschallechosignalen, die durch Reflektieren der Ultraschallsignale von dem Objekt (10) erhalten werden; und
Durchführen einer Strahlformung an den empfangenen Ultraschallechosignalen,
wobei das Durchführen der Strahlformung der empfangenen Ultraschallechosignale Folgendes umfasst:

> Einstellen (S710), basierend auf dem Standort des Brennpunkts (410), einer Vielzahl von Verzögerungszeiten, die jeweils der Vielzahl von Elementen entsprechen; Bestimmen (S730) der Anzahl von für die Zeitverzögerung zu verwendenden Kondensatoren (320), die jeweils der Vielzahl von Elementen entsprechen, basierend auf der eingestellten Vielzahl von Verzögerungszeiten, so dass eine Zeit, in der ein Signal nicht in einem für die Zeitverzögerung verwendeten Kondensator (320) gespeichert ist, kleiner als eine vorbestimmte Zeit ist; und
> jeweiliges Durchführen (S750) der Strahlformung an den empfangenen Ultraschallechosignalen unter Verwendung der bestimmten Anzahl von Kondensatoren (320).

9. Ultraschallbildgebungsvorrichtung (100), die Folgendes umfasst:

> einen Prozessor (120), der zum Einstellen eines Brennpunkts in einem Objekt (10) konfiguriert ist;
> einen Wandler (110), der eine Vielzahl von Elementen enthält und so konfiguriert ist, dass er Ultraschallsignale in das Objekt (10) basierend auf einem Standort des Brennpunkts (410) überträgt und Ultraschallechosignale empfängt, die durch Reflexion der Ultraschallsignale von dem Objekt (10) erhalten werden; und
> eine Vielzahl von Kondensatoren (420), die verwendet werden, um eine Strahlformung an den empfangenen Ultraschallechosignalen durchzuführen,
> wobei der Prozessor (120) zu Folgendem konfiguriert ist:

> Einstellen (S710), basierend auf dem Standort des Brennpunkts (410), einer Vielzahl von Verzögerungszeiten, die jeweils der Vielzahl von Elementen entsprechen; Bestimmen (S730) der Anzahl von für die Zeitverzögerung zu verwendenden Kondensatoren (320), die jeweils der Vielzahl von Elementen entsprechen, basierend auf der eingestellten Vielzahl von Verzögerungszeiten, so dass eine Zeit, in der ein Signal nicht in einem für die Zeitverzögerung verwendeten Kondensator (320) gespeichert ist, kleiner als eine vorbestimmte Zeit ist; und
> jeweiliges Durchführen (S750) der Strahlformung an den empfangenen Ultraschallechosignalen unter Verwendung der bestimmten Anzahl von Kondensatoren (320).

10. Ultraschallbildgebungsvorrichtung (100) nach Anspruch 9, wobei der Prozessor (120) ferner so konfiguriert ist, dass er die Anzahl der Kondensatoren (320), die jedem aus der Vielzahl von Elementen entspricht, durch Vergleichen jeder aus der eingestellten Vielzahl von Verzögerungszeiten mit einer voreingestellten Referenzzeit bestimmt (S1210).

11. Ultraschallbildgebungsvorrichtung (100) nach Anspruch 10, wobei der Prozessor (120) ferner zu Folgendem konfiguriert ist:

> Bestimmen (S1230) eines Bereichs, der jeder aus der eingestellten Vielzahl von Verzögerungszeiten entspricht, aus einer Vielzahl von Bereichen, die durch Klassifizierung basierend auf der voreingestellten Referenzzeit erhalten werden, durch Vergleichen jeder aus der eingestellten Vielzahl von Verzögerungszeiten mit der voreingestellten Referenzzeit; und
> Bestimmen (S1250) der Anzahl der Kondensatoren (320), die dem bestimmten Bereich entsprechen.

12. Ultraschallbildgebungsvorrichtung (100) nach Anspruch 9, wobei der Prozessor (120) ferner so konfiguriert ist, dass er die Anzahl der Kondensatoren (320), die jedem aus der Vielzahl von Elementen entspricht, durch Vergleichen einer längsten Verzögerungszeit aus der eingestellten Vielzahl von Verzögerungszeiten mit jeder der eingestellten Vielzahl von Verzögerungszeiten bestimmt.

13. Ultraschallbildgebungsvorrichtung (100) nach Anspruch 9, wobei die Anzahl der Vielzahl von Elementen, die die gleiche bestimmte Anzahl von Kondensatoren (320) aufweist, kleiner oder gleich einem vorbestimmten Wert ist.

14. Ultraschallbildgebungsvorrichtung (100) nach Anspruch 9, wobei die Anzahl der Arten einer Kategorie, die die bestimmte Anzahl von Kondensatoren angibt, größer oder gleich einem vorbestimmten Wert ist.

15. Ultraschallbildgebungsvorrichtung (100) nach Anspruch 9, wobei der Prozessor (120) ferner so konfiguriert ist, dass er die strahlgeformten Ultraschallechosignale in Ultraschallbildsignale umwandelt, wobei die Ultraschallbildgebungsvorrichtung (100) ferner eine Anzeige (140) umfasst, die die Ultraschallbildsignale anzeigt.

**Revendications**

1. Procédé de génération d'une image échographique, le procédé comprenant les étapes consistant à :

   définir un point focal dans un objet (10) ;
   émettre, compte tenu de l'emplacement du point focal (410), des signaux ultrasonores vers l'objet (10) au moyen d'une pluralité d'éléments de transducteur (420) ;
   recevoir, par le biais de la pluralité d'éléments (420), des signaux d'écho ultrasonores obtenus par réflexion des signaux ultrasonores par l'objet (10) ; et
   réaliser une mise en forme de faisceaux sur les signaux d'écho ultrasonores reçus ;
   ladite réalisation de la mise en forme de faisceaux des signaux d'écho ultrasonores reçus comprenant :

   la définition (S710), compte tenu de l'emplacement du point focal (410), d'une pluralité de temps de retard correspondant respectivement à la pluralité d'éléments ;
   la détermination (S730) du nombre de condensateurs (320) à utiliser pour le retard et correspondant respectivement à la pluralité d'éléments compte tenu de la pluralité de temps de retard définis, de telle sorte qu'un temps pendant lequel un signal n'est pas stocké dans un condensateur (320) utilisé pour le retard soit inférieur à un temps prédéterminé ; et
   la réalisation (S750) de la mise en forme de faisceaux sur les signaux d'écho ultrasonores reçus respectifs au moyen du nombre déterminé de condensateurs (320).

2. Procédé selon la revendication 1, dans lequel le nombre de condensateurs (320) correspondant à chaque élément de la pluralité d'éléments est déterminé par comparaison de chaque temps de retard de la pluralité de temps de retard définis avec un temps de référence prédéfini (S1210).

3. Procédé selon la revendication 2, dans lequel la détermination du nombre de condensateurs (320) comprend :

   la détermination (S1230) d'une plage, correspondant à chaque temps de retard de la pluralité de temps de retard définis parmi une pluralité de plages obtenues à la suite d'un classement en fonction du temps de référence prédéfini, par comparaison de chaque temps de retard de la pluralité de temps de retard définis avec le temps de référence prédéfini, et
   la détermination (S1250) du nombre de conden-

sateurs (320) correspondant à la plage déterminée.

4. Procédé selon la revendication 1, dans lequel le nombre de condensateurs (320) correspondant à chaque élément de la pluralité d'éléments est déterminé par comparaison du temps de retard le plus long parmi la pluralité de temps de retard définis avec chaque temps de retard de la pluralité de temps de retard définis.

5. Procédé selon la revendication 1, dans lequel le nombre d'une pluralité d'éléments ayant le même nombre déterminé de condensateurs (320) est inférieur ou égal à une valeur prédéterminée.

6. Procédé selon la revendication 1, dans lequel le nombre de types d'une catégorie indiquant le nombre déterminé de condensateurs (320) est supérieur ou égal à une valeur prédéterminée.

7. Procédé selon la revendication 1, comprenant en outre les étapes consistant à :

   convertir les signaux d'écho ultrasonores formés en faisceaux en signaux d'image échographique, et
   afficher les signaux d'image échographique.

8. Produit-programme d'ordinateur comprenant un support de stockage (150) lisible par ordinateur comprenant des instructions qui, lorsqu'elles sont exécutées par un processeur (120) d'un appareil échographique (100), amènent le processeur (120) à :

   définir un point focal dans un objet (10) ;
   émettre, compte tenu de l'emplacement du point focal (410), des signaux ultrasonores vers l'objet (10) au moyen d'une pluralité d'éléments de transducteur (420) ;
   recevoir, par le biais de la pluralité d'éléments, des signaux d'écho ultrasonores obtenus par réflexion des signaux ultrasonores par l'objet (10) ; et
   réaliser une mise en forme de faisceaux sur les signaux d'écho ultrasonores reçus ;
   ladite réalisation de la mise en forme de faisceaux des signaux d'écho ultrasonores reçus comprenant :

   la définition (S710), compte tenu de l'emplacement du point focal (410), d'une pluralité de temps de retard correspondant respectivement à la pluralité d'éléments ;
   la détermination (S730) du nombre de condensateurs (320) à utiliser pour le retard et correspondant respectivement à la pluralité d'éléments compte tenu de la pluralité de temps de retard définis, de telle sorte qu'un temps pendant lequel un signal n'est pas stocké dans un condensateur (320) utilisé pour le retard soit inférieur à un temps prédéterminé ; et

la réalisation (S750) de la mise en forme de faisceaux sur les signaux d'écho ultrasonores reçus respectifs au moyen du nombre déterminé de condensateurs (320).

9. Appareil échographique (100) comprenant :

un processeur (120) conçu pour définir un point focal dans un objet (10) ;

un transducteur (110) comprenant une pluralité d'éléments et conçu pour émettre des signaux ultrasonores vers l'objet (10) compte tenu de l'emplacement du point focal (410) et pour recevoir des signaux d'écho ultrasonores obtenus par réflexion des signaux ultrasonores par l'objet (10) ; et une pluralité de condensateurs (420) utilisés pour réaliser une mise en forme de faisceaux sur les signaux d'écho ultrasonores reçus ;

ledit processeur (120) étant conçu pour :

définir (S710), compte tenu de l'emplacement du point focal (410), une pluralité de temps de retard correspondant respectivement à la pluralité d'éléments ;

déterminer (S730) le nombre de condensateurs (320) à utiliser pour le retard et correspondant respectivement à la pluralité d'éléments compte tenu de la pluralité de temps de retard définis, de telle sorte qu'un temps pendant lequel un signal n'est pas stocké dans un condensateur (320) utilisé pour le retard temporel soit inférieur à un temps prédéterminé ; et

réaliser (S750) la mise en forme de faisceaux sur les signaux d'écho ultrasonores reçus respectifs au moyen du nombre déterminé de condensateurs (320).

10. Appareil échographique (100) selon la revendication 9, dans lequel le processeur (120) est en outre conçu pour déterminer le nombre de condensateurs (320) correspondant à chaque élément de la pluralité d'éléments par comparaison de chaque temps de retard de la pluralité de temps de retard définis avec un temps de référence prédéfini (S1210).

11. Appareil échographique (100) selon la revendication 10, dans lequel le processeur (120) est en outre conçu pour :

déterminer (S 1230) une plage, correspondant à chaque temps de retard de la pluralité de temps de retard définis parmi une pluralité de plages obtenues à la suite d'un classement en fonction du temps de référence prédéfini, par comparaison de chaque temps de retard de la pluralité de temps de retard définis avec le temps de référence prédéfini, et

déterminer (S 1250) le nombre de condensateurs (320) correspondant à la plage déterminée.

12. Appareil échographique (100) selon la revendication 9, dans lequel le processeur (120) est en outre conçu pour déterminer le nombre de condensateurs (320) correspondant à chaque élément de la pluralité d'éléments par comparaison du temps de retard le plus long parmi la pluralité de temps de retard définis avec chaque temps de retard de la pluralité de temps de retard définis.

13. Appareil échographique (100) selon la revendication 9, dans lequel le nombre d'une pluralité d'éléments ayant le même nombre déterminé de condensateurs (320) est inférieur ou égal à une valeur prédéterminée.

14. Appareil échographique (100) selon la revendication 9, dans lequel le nombre de types d'une catégorie indiquant le nombre déterminé de condensateurs est supérieur ou égal à une valeur prédéterminée.

15. Appareil échographique (100) selon la revendication 9, dans lequel le processeur (120) est en outre conçu pour convertir les signaux d'écho ultrasonore formés en faisceaux en signaux d'image échographique, l'appareil échographique (100) comprenant en outre un écran (140) affichant les signaux d'image échographique.

# FIG. 1

EP 3 782 553 B1

# FIG. 2A

# FIG. 2B

FIG. 2C

100c

145

40

20

# FIG. 3

# FIG. 4

# FIG. 5

# FIG. 6

EP 3 782 553 B1

# FIG. 7

START

SET PLURALITY OF DELAY TIMES RESPECTIVELY CORRESPONDING TO PLURALITY OF ELEMENTS — S710

DETERMINE CAPACITOR CORRESPONDING TO EACH OF PLURALITY OF ELEMENTS — S730

PERFORM BEAMFORMING ON ULTRASOUND ECHO SIGNAL BY USING DETERMINED CAPACITOR — S750

END

# FIG. 8A

$\Phi_1$

$\Phi_2$

$\Phi_3$

$\Phi_4$

$\Phi_5$

$\Phi_6$

$\Phi_7$

$\Phi_8$

$\Phi_{1d}$

$\Phi_{2d}$

$\Phi_{3d}$

$\Phi_{4d}$

$\Phi_{5d}$

$\Phi_{6d}$

$\Phi_{7d}$

$\Phi_{8d}$

$\tau = 1 \cdot \Delta t$

# FIG. 8B

$\tau = 2 \cdot \Delta t$

# FIG. 8C

$\tau = 3 \cdot \Delta t$

# FIG. 8D

$\tau = 4 \cdot \Delta t$

# FIG. 8E

# FIG. 8F

$\tau = 6 \cdot \Delta t$

# FIG. 8G

$\tau = 7 \cdot \Delta t$

# FIG. 9

Vout
$\tau = (1 \cdot \Delta t)$
$2 \cdot \Delta t$

Vout
$\tau = (2 \cdot \Delta t)$
$3 \cdot \Delta t$

Vout
$\tau = (3 \cdot \Delta t)$
$4 \cdot \Delta t$

Vout
$\tau = (4 \cdot \Delta t)$
$5 \cdot \Delta t$

Vout
$\tau = (5 \cdot \Delta t)$
$6 \cdot \Delta t$

Vout
$\tau = (6 \cdot \Delta t)$
$7 \cdot \Delta t$

Vout
$\tau = (7 \cdot \Delta t)$
$8 \cdot \Delta t$

# FIG. 10

# FIG. 11

| Delaytime/s | ECHO | Harmonic frequency (kH/z) | Delaytime/s | ECHO | Harmonic frequency (kH/z) | Delaytime/s | ECHO | Harmonic frequency (kH/z) | Delaytime/s | ECHO | Harmonic frequency (kH/z) | Delaytime/s | ECHO | Harmonic frequency (kH/z) |
|---|---|---|---|---|---|---|---|---|---|---|---|---|---|---|
| 25n | 2 | 20000 | 500n | 21 | 1905 | 1000n | 41 | 976 | 1500n | 61 | 656 | 2000n | 81 | 494 |
| 50n | 3 | 13333 | 525n | 22 | 1818 | 1025n | 42 | 952 | 1525n | 62 | 645 | 2025n | 82 | 488 |
| 75n | 4 | 10000 | 550n | 23 | 1739 | 1050n | 43 | 930 | 1550n | 63 | 635 | 2050n | 83 | 482 |
| 100n | 5 | 8000 | 575n | 24 | 1667 | 1075n | 44 | 909 | 1575n | 64 | 625 | 2075n | 84 | 476 |
| 125n | 6 | 6667 | 600n | 25 | 1600 | 1100n | 45 | 889 | 1600n | 65 | 615 | 2100n | 85 | 471 |
| 150n | 7 | 5714 | 625n | 26 | 1538 | 1125n | 46 | 870 | 1625n | 66 | 606 | 2125n | 86 | 465 |
| 175n | 8 | 5000 | 650n | 27 | 1481 | 1150n | 47 | 851 | 1650n | 67 | 597 | 2150n | 87 | 460 |
| 200n | 9 | 4444 | 675n | 28 | 1429 | 1175n | 48 | 833 | 1675n | 68 | 588 | 2175n | 88 | 455 |
| 225n | 10 | 4000 | 700n | 29 | 1379 | 1200n | 49 | 816 | 1700n | 69 | 580 | 2200n | 89 | 449 |
| 250n | 11 | 3636 | 725n | 30 | 1333 | 1225n | 50 | 800 | 1725n | 70 | 571 | 2225n | 90 | 444 |
| 275n | 12 | 3333 | 750n | 31 | 1290 | 1250n | 51 | 784 | 1750n | 71 | 563 | 2250n | 91 | 440 |
| 300n | 13 | 3077 | 775n | 32 | 1250 | 1275n | 52 | 769 | 1775n | 72 | 556 | 2275n | 92 | 435 |
| 325n | 14 | 2857 | 800n | 33 | 1212 | 1300n | 53 | 755 | 1800n | 73 | 548 | 2300n | 93 | 430 |
| 350n | 15 | 2667 | 825n | 34 | 1176 | 1325n | 54 | 741 | 1825n | 74 | 541 | 2325n | 94 | 426 |
| 375n | 16 | 2500 | 850n | 35 | 1143 | 1350n | 55 | 727 | 1850n | 75 | 533 | 2350n | 95 | 421 |
| 400n | 17 | 2353 | 875n | 36 | 1111 | 1375n | 56 | 714 | 1875n | 76 | 526 | 2375n | 96 | 417 |
| 425n | 18 | 2222 | 900n | 37 | 1081 | 1400n | 57 | 702 | 1900n | 77 | 519 | 2400n | 97 | 412 |
| 450n | 19 | 2105 | 925n | 38 | 1053 | 1425n | 58 | 690 | 1925n | 78 | 513 | 2425n | 98 | 408 |
| 475n | 20 | 20000 | 950n | 39 | 1026 | 1450n | 59 | 678 | 1950n | 79 | 506 | 2450n | 99 | 404 |
|  |  |  | 975n | 40 | 1000 | 1475n | 60 | 667 | 1975n | 80 | 500 | 2475n | 100 | 400 |

EP 3 782 553 B1

# FIG. 12

START

CLASSIFY PRESET REFERENCE TIMES INTO PLURALITY OF RANGES — S1210

DETERMINE A RANGE CORRESPONDING TO EACH OF PLURALITY OF DELAY TIMES — S1230

DETERMINE NUMBER OF CAPACITORS CORRESPONDING TO DETERMINED RANGE — S1250

END

# FIG. 13

START

DETERMINE NUMBER OF CAPACITORS
CORRESPONDING TO DETERMINED RANGE ——— S1310

CLASSIFY DELAY TIME INTO PLURALITY OF
RANGES BASED ON SET REFERENCE TIME ——— S1330

DETERMINE RANGE CORRESPONDING TO
EACH OF PLURALITY OF DELAY TIMES ——— S1350

DETERMINE NUMBER OF CAPACITORS
CORRESPONDING TO DETERMINED RANGE ——— S1370

END

**EP 3 782 553 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018008234 A1